Europäisches Patentamt

**European Patent Office**

Office européen des brevets

(11) Numéro de publication: **0 317 401**
A1

(12) **DEMANDE DE BREVET EUROPEEN**

(21) Numéro de dépôt: **88402829.1**

(22) Date de dépôt: **10.11.88**

(51) Int. Cl.⁴: **A 61 K 31/475**
A 61 K 9/08, A 61 K 47/00

(30) Priorité: **13.11.87 FR 8715686**

(43) Date de publication de la demande:
**24.05.89 Bulletin 89/21**

(84) Etats contractants désignés:
**AT BE CH DE ES GB GR IT LI LU NL SE**

(71) Demandeur: **PIERRE FABRE MEDICAMENT**
**125, Rue de la Faisanderie**
**F-75116 Paris (FR)**

(72) Inventeur: **Leverd, Elie**
**Chemin de Cazers Bas Lambert**
**F-81100 Castres (FR)**

**Bauer, Michel**
**150 Chemin des Fourches Hautes**
**F-81100 Castres (FR)**

**Basquin, Serge**
**14, rue Borrel**
**F-81100 Castres (FR)**

(74) Mandataire: **Ahner, Francis et al**
**CABINET REGIMBEAU 26, avenue Kléber**
**F-75116 Paris (FR)**

(54) **Composition pharmaceutique pour l'administration parentérale de navelbine.**

(57) La présente invention concerne une composition pharmaceutique pour l'administration parentérale de navelbine, caractérisée en ce qu'elle se présente sous la forme d'une solution aqueuse injectable stable prête à l'emploi.

EP 0 317 401 A1

Bundesdruckerei Berlin

Description

# COMPOSITION PHARMACEUTIQUE POUR L'ADMINISTRATION PARENTERALE DE NAVELBINE

La présente invention concerne une composition pharmaceutique pour l'administration parentérale de navelbine.

L'étude des propriétés antinéoplasiques des alcaloïdes de VINCA ROSEA (famille des Apocynacées) a permis de mettre en évidence les activités intéressantes de la vinblastine et de la vincristine.

La navelbine est un dérivé particulièrement efficace de la vinblastine : il s'agit de la Nor-5'-anhydro-vinblastine dont la synthèse est à présent connue (FR-A-2 448 545).

Actuellement, la chimiothérapie des cancers fait appel à l'emploi associé de plusieurs oncostatiques ayant des toxicités et des modes d'action différents. En outre, de trop nombreuses chimiothérapies se soldent par des échecs dus probablement à l'insuffisance ou l'inefficacité des produits empolyés. Devant la recrudescence actuelle des cas de cancers, les besoins en produits oncostatiques sont énormes.

C'est pourquoi la présente invention concerne une composition pharmaceutique pour l'administration parentérale et, notamment intraveineuse; de navelbine, caractérisée en ce qu'elle se présente sous la forme d'une solution injectable stable prête à l'emploi.

La stabilité particulière de la solution injectable selon la présente invention est obtenue au moyen d'une formulation stérile comportant un sel hydrosoluble de navelbine, un agent osmotique, un agent conservateur anti-microbien et de l'eau pour préparation injectable.

De préférence, selon la présente invention, le sel hydrosoluble de navelbine est le di-tartrate. La quantité de principe actif varie bien sûr en fonction de l'état du patient et de l'appréciation du médecin. Elle est en moyenne comprise entre 10 et 50 mg de navelbine base par dose unitaire.

L'agent osmotique de la solution injectable parentérale de la présente invention doit conférer à cette dernière une pression osmotique sensiblement égale à celle du plasma sanguin. Cet agent osmotique comporte, de préférence, une ou plusieurs substances choisies parmi les électrolytes, tels que le chlorure de sodium, les acides aminés, tels que le glycocolle, les sucres, tels que le glucose et/ou les polyols, tels que la mannitol. Ces substances sont utilisées à une concentration de préférence inférieure ou égale à la concentration d'iso-osmose; ainsi le pourcentage de mannitol utilisé est environ égal à 5,07 % (P/V); celui du glucose est environ égal à 5,05 % (P/V).

La stérilité de la composition selon l'invention est assurée grâce au conservateur anti-microbien; ce dernier comporte, de préférence, une ou plusieurs substances choisies parmi les esters ou les sels de l'acide p-hydroxybenzoïque, l'alcool benzylique, les composés mercuriels tels que le mercurothiolate sodique, le chlorobutanol et/ou le phénol ou ses dérivés.

Ces substances peuvent être utilisées seules ou en association entre elles; en tout état de cause, ces substances sont utilisées en quantité efficace et pharmacologiquement compatible; c'est pourquoi des teneurs maximales d'utilisation sont prévues pour chacune d'entre elles :

Ces teneurs, exprimées en P/V sont de 1,5 %₀ pour les dérivés de l'acide parahydroxybenzoïque, 10 %₀ pour l'alcool benzylique, 0,1 %₀ pour les composés mercuriels et 5 %₀ pour le chlorobutanol ou les dérivés du phénol.

La présence d'agent anti-microbien permet de protéger la formulation de tout risque de contamination microbienne accidentelle lors de sa fabrication. De plus, elle autorise la distribution de la solution injectable sous forme de conditionnement multidose.

En outre en raison de la thermosensibilité des sels de navelbine en solution aqueuse, la stérilisation de la solution est assurée par filtration sur une membrane de porosité 0,2 μm.

A titre d'exemple, la composition de la présente invention peut répondre à la formule suivante :

| . Di-tartrate de Navelbine... | 13,85 mg |
|---|---|
| . Phénol... | 5 mg |
| . Mannitol... | 37,20 mg |
| . Eau pour préparations injectables... qsp... | 1 ml |

Le récipient de conditionnement peut être choisi parmi les ampoules de verre blanc ou brun ou les flacons en verre munis d'un bouchon en élastomère.

Des essais relatifs à la stabilité de la solution selon la présente invention ont été effectués et sont présentés dans l'exemple suivant illustré par les figures 1, 2 et 3 qui représentent la chromatogramme de la solution aux temps et températures suivants :

    Fig. 1 - temps 0
    Fig. 2 - temps = 6 semaines et température ambiante
    Fig. 3 - temps = 6 semaines et température = 40°C

## Exemple

\* - Etude analytique de la stabilité de la solution
    La composition répondant à la formule suivante

| . Navelbine di-tartrate... | 13,85 mg |
|---|---|
| . Phénol... | 5 mg |
| . Mannitol... | 37,20 mg |
| . Eau pour préparations injectables... qsp... | 1 ml |

a été maintenue dans un récipient de verre à température ambiante et à 40°C, pendant 6 semaines. Une analyse par chromatographie liquide haute performance (CLHP) a été effectuée pour le dosage spécifique de la NAVELBINE. La méthode CLHP a été la suivante :

- appareil : chromatographe en phase liquide muni d'un détecteur à longueur d'onde variable, fixée ici à 267 nm et d'un injecteur automatique.
- colonne Lichrocart L = 12 cm $C_{18}$ - 5 μm (Merck)
- phase éluante : méthanol 800 ml
tampon 100 ml
phosphate
pH = 7,5
eau 100 ml

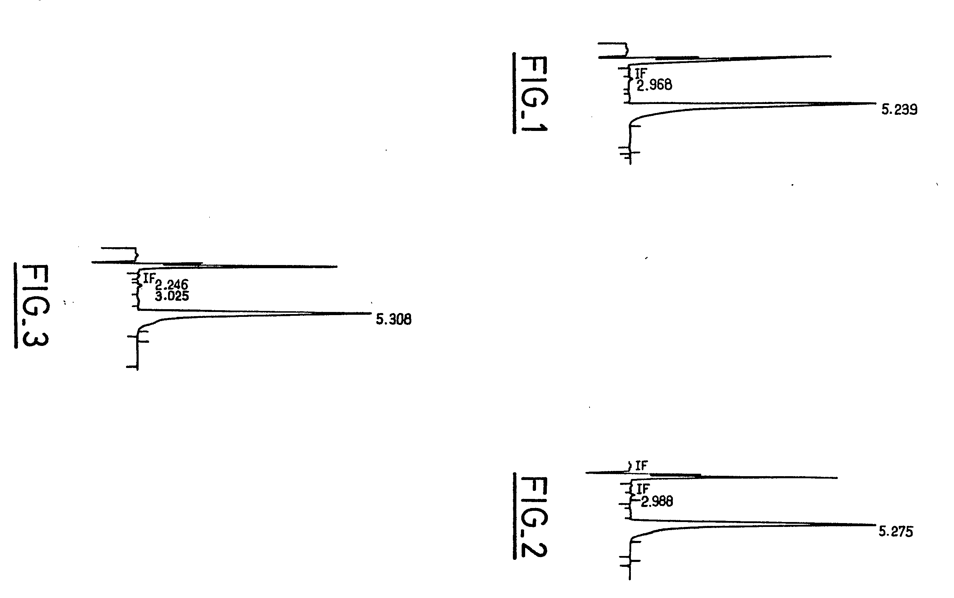

On peut remarquer, à partir des figures 1, 2 et 3 qui représentent les chromatogrammes de la solution pour t = 0 et t = 6 semaines à température ambiante et 40°C, que la teneur en navelbine après 6 semaines à 40°C n'est pas inférieure à 97 p.cent de la valeur initiale.

**Revendications**

1 - Composition pharmaceutique pour l'administration parentérale de navelbine caractérisée en ce qu'elle se présente sous la forme d'une solution aqueuse injectable stable prête à l'emploi.

2 - Composition selon la revendication 1 ,caractérisée en ce qu'elle comporte un sel hydrosoluble de navelbine, un agent osmotique, un agent conservateur anti-microbien et de l'eau pour préparation injectable.

3 - Composition selon la revendication 2 ,caractérisée en ce que le sel de navelbine est le di-tartrate.

4 - Composition selon la revendication 2 ou 3, caractérisée en ce que l'agent osmotique comprend une ou plusieurs substances choisies parmi les électrolytes, tels que le chlorure de sodium, les acides aminés, tels que le glycocolle, les sucres, tels que le glucose et/ou les polyols, tels que le mannitol.

5 - Composition selon l'une quelconque des revendications 2 à 4,caractérisée en ce que l'agent conservateur antimicrobien comprend une ou plusieurs substances choisies parmi les esters ou les sels de l'acide p-hydroxybenzoïque, l'alcool benzylique, les composés mercuriels tels que le mercurothiolate sodique, le chlorobutanol et/ou le phénol ou ses dérivés.

6 - Composition selon l'une quelconque des revendications 1 à 5,caractérisée en ce qu'elle répond à la formule suivante :

| . Di-tartrate de navelbine... | 13,85 mg |
| . Phénol... | 5 mg |
| . Mannitol... | 37,20 mg |
| . Eau pour préparations injectables... qsp... | 1 ml |

FIG_1

FIG_2

FIG_3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 243 278  (PIERRE FABRE MEDICAMENT) <br> * Page 1, ligne 25 - page 3, ligne 17; revendications 1-3 * <br> --- | 1-6 | A 61 K  31/475 <br> A 61 K   9/08 <br> A 61 K  47/00 |
| Y | DE-A-3 324 964  (ELI LILLY & CO.) <br> * Revendications 1-13 * <br> --- | 1-6 | |
| Y | FR-A-2 571 724  (RICHTER GEDEON VEGYESZETI GYAR R.T.) <br> * @Exemples 1-7 * <br> --- | 1-6 | |
| A | GB-A-2 020 180  (NELSON RESEARCH & DEVELOPMENT CO.) <br> * Page 1, ligne 30 - page 2, ligne 7 * <br> --- | | |
| D,A | FR-A-2 448 545  (AGENCE NATIONALE DE VALORISATION DE LA RECHERCHE (ANVAR)) <br> * Revendication 1 * <br> ----- | | |

**DOMAINES TECHNIQUES RECHERCHES (Int. Cl.4)**

A 61 K

Le présent rapport a été établi pour toutes les revendications

| Lieu de la recherche | Date d'achèvement de la recherche | Examinateur |
|---|---|---|
| LA HAYE | 23-01-1989 | TZSCHOPPE,D.A. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
................................................................
& : membre de la même famille, document correspondant

EPO FORM 1503 03.82 (P0402)